# EUROPEAN PATENT APPLICATION

(11) **EP 1 826 204 A1**
(43) Date of publication of application: **29.08.2007**
(21) Application number: 06380038.7
(22) Date of filing: 28.02.2006
(51) Int. Cl.: C07D 333/12, C07D 333/14, C07D 333/24

(54) **Process for obtaining enantiomers of duloxetine precursors**

(71) Applicant: LABORATORIOS DEL DR. ESTEVE, S.A., 08041 Barcelona (ES)
(72) Inventor: Torrens Jover, Antoni, 08041 Barcelona (ES); Buschmann, Helmut Henrich, 08950 Esplugues de Llobregat Barcelona (ES); Dahmen, Stefan, 52074 Aachen (DE); Lormann, Matthias, 52072 Aachen (DE)
(74) Representative: ABG Patentes, S.L.

(57) **Abstract**

The present invention is directed to a process for the preparation of an enantiomerically enriched compound of formula II 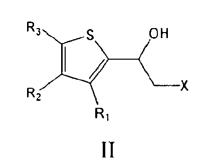 wherein,
R₁, R₂ and R₃ are each independently selected from hydrogen, halogen, substituted or unsubstituted lower alkyl or substituted or unsubstituted aryl;
X is -C(=O)-Z or -Y, wherein -Y is selected from -CH₂-OR₄, -CH₂-halogen or - CH₂-NR₆R₇;
wherein
Z is selected from -NR₆R₇ or -OR₅, wherein R₅ is selected from hydrogen, substituted or unsubstituted lower alkyl or ester activating group;
R₄ is selected from hydrogen, hydroxyl protecting group or hydroxyl activating group;
R₆ and R₇ are each independently selected from hydrogen, amino protecting group, amido protecting group or substituted or unsubstituted lower alkyl;

which comprises an enantioselective addition with a thienyl zinc reagent, in the presence of a chiral ligand.

## Description

### FIELD OF THE INVENTION

The present invention relates to a new process for the preparation of enantiomerically enriched thienylalcoxypropaneamines. Said compounds are useful intermediates for the preparation of Duloxetine.

### BACKGROUND OF THE INVENTION

The compound (±)-N-Methyl-N-[3-(naphthalene-1-yloxy)-3-(2-thienyl)propyl]amine, also known as Duloxetine, of formula I 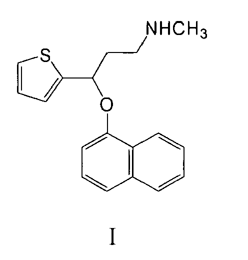 is a potent 5-HT reuptake and norepinephrine reuptake inhibitor which has been already launched to the market for the treatment of different conditions (depression, urinary incontinence and neuropathic pain). Duloxetine is also under clinical trials for the treatment of other conditions such as generalized anxiety and fibromyalgia. Of the two possible enantiomers of Duloxetine, the dextrorotatory enantiomer, (+)-Duloxetine, is more potent than the (-)-Duloxetine.

Duloxetine can be prepared by O-alkylation of a compound of formula II:

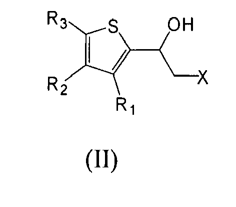

The pure enantiomers of (+)-I and (-)-1 may be prepared by separately O-alkylating the enantiomerically pure intermediates (+)-II and (-)-II. Thus, a synthetic process to the enantiomerically pure/enriched intermediates (+)-II and (-)-II is needed.

The enantioselective reduction of prochiral ketones has been proposed in organic synthesis to obtain secondary alcohols with high enantiomeric purity. Accordingly, a number of strategies for the asymmetric reduction of prochiral ketones to enantiomerically enriched alcohols have been developed [R. Noyori, T. Ohkuma, Angew. Chem. Int. Ed., 2001, 40, 40-73, Wiley-VCH Verlag].

Also, in patent application n° CA 2498756 compounds of formula II are synthesized by acylating an acetyl thiophene of formula (1) 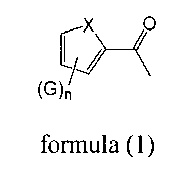 and subsequent asymmetric hydrogenation in the presence of a chiral catalyst. However, this method requires two steps in order to arrive at the compound of formula II. Additionally, the reagents needed (for example, NaH) are not compatible with industrial production.

In patent application n° DE 10237272 a thiophenecarbaldehyde derivative is subjected to a Reformatsky type reaction.

A phenyl transfer reaction to aryl aldehydes as an approach towards enantio-pure diarylalcohols has also been proposed, as alternative to the enantioselective reduction of prochiral ketones [P.I. Dosa, J.C. Ruble, G.C. Fu, J. Org. Chem. 1997, 62 444; W.S. Huang, L. Pu, Tetrahedron Lett. 2000, 41, 145; M. Fontes, X.Verdaguer, L. Solà, M.A. Pericàs, A. Riera, J. Org. Chem. 2004, 69, 2532]. For this transformation, the group of Bolm et al. developed a protocol which utilized a ferrocene-based ligand (or catalyst) and diphenylzinc in combination with diethylzinc as aryl source [C. Bolm, N. Hermanns, M. Kesselgruber, J.P. Hildebrand, J. Organomet. Chem. 2001, 624, 157; C. Bolm, N. Hermanns, A. Claßen, K. Muñiz, Bioorg. Med. Chem. Lett. 2002, 12,1795]. Enantiomerically enriched diarylmethanols with excellent enantiomeric excess (up to 99% ee) were thus obtained in a straightforward manner. Subsequently, the applicability of air-stable arylboronic acids as aryl source was also demonstrated [C. Bolm, J. Rudolph, J. Am. Chem. Soc. 2002, 124, 14850]. However, these systems require a high catalyst loading (of commonly 10% mol.) to achieve that high enantioselectivity. With the aim of reducing this problem, recently, it has been proposed the use of triphenylborane as an alternative phenyl source in a reaction where the ferrocene-based catalyst is also used (J. Rudolph, F. Schmidt, C. Bolm, Adv. Synth. Catal. 2004, 346, 867). It has been applied with difficulties to heteroaromatic aldehydes, for example to 2-thiophenecarbaldehyde.

However, there are still some difficulties to obtain chiral alcohols with a high yield and enantioselectivity without a high amount of catalyst. For their large-scale preparation, the application of highly efficient catalytic system and enantioselective methods employing inexpensive starting materials and simple purification steps would be most desirable.

Additionally, the application of these processes so as to transfer a heteroaryl moiety to an aldehyde is challenging. Most examples described in the literature are directed to the phenyl transfer to a benzaldehyde derivative or to an alkyl-aldehyde derivative. There is at the present time no example of an enantioselective addition of thienyl reagents to β-substitutedaldehydes.

Thus, to attain satisfactory ee values by an enantioselective addition reaction, an appropriate coordination of the catalyst system and the aldehyde is required. The results with unusual substrates cannot be predicted and each addition has to be investigated separately with regard to the substrate.

### SUMMARY OF THE INVENTION

We have now surprisingly found that β-substitutedaldehydes can be successfully used as substrates for a thienyl transfer reaction. We have therefore applied this process to the synthesis of the enantiomerically pure intermediates of compounds of formula II and to a process to obtain N-Methyl-N-[3-(naphthalene-1-yloxy)-3-(2-thienyl)propyl]amine and in general to thienylalcoxypropaneamines and their enantiomers. This process is specially useful for the industrial scale; it provides high enantiomeric excess with less quantity of catalyst. Additionally, raw materials are costs-effective. Further, heavy metals are not used, avoiding the presence of potentially toxic impurities. Another advantage is that impurities generated during the process are easily eliminated.

Accordingly, the present invention refers to a process for the asymmetric addition of a thienyl group to a β-substituted aldehyde by means of a thienyl zinc reagent in the presence of a chiral ligand. Said process allows the preparation of known intermediates of formula (II), which thereafter can yield, by O-alkylation, the desired enantiomers of pharmaceutically active thienylalcoxypropaneamines, particularly the pharmaceutically active compound N-Methyl-N-[3-(naphthalene-1-yloxy)-3-(2-thienyl)propyl]amine.

Therefore, according to one aspect, the present invention is directed to a process for the preparation of an enantiomerically enriched compound of formula II 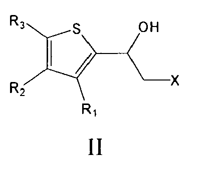 wherein,
R₁, R₂ and R₃ are each independently selected from hydrogen, halogen, substituted or unsubstituted lower alkyl or substituted or unsubstituted aryl;
X is -C(=O)-Z or Y, wherein Y is selected from -CH₂-OR₄, -CH₂-halogen or - CH₂-NR₆R₇;
wherein
Z is selected from -NR₆R₇ or -OR₅, wherein R₅ is selected from hydrogen, substituted or unsubstituted lower alkyl or ester activating group;
R₄ is selected from hydrogen, hydroxyl protecting group or hydroxyl activating group;
R₆ and R₇ are each independently selected from hydrogen, amino protecting group, amido protecting group or substituted or unsubstituted lower alkyl;
which comprises an enantioselective addition reaction to a compound of formula III 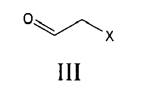 wherein X has the same meaning as above;
with a thienyl zinc reagent optionally substituted on the thienyl ring, in the presence of a chiral ligand.

In a preferred embodiment the present invention is directed to a process for the preparation of an enantiomerically enriched compound of formula IIa 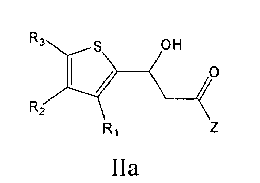 wherein,
R₁, R₂ and R₃ and Z have the same meaning as above,
which comprises an enantioselective addition reaction to a compound of formula IIIa 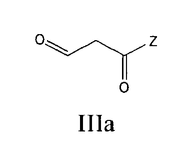 wherein Z has the same meaning as above;
with a thienyl zinc reagent optionally substituted on the thienyl ring, in the presence of a chiral ligand.

In another preferred embodiment the present invention is directed to a process for the preparation of an enantiomerically enriched compound of formula IIb 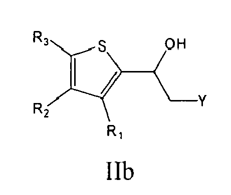 wherein,
R₁, R₂ and R₃ and Y have the same meaning as above;
which comprises an enantioselective addition reaction to a compound of formula IIIb 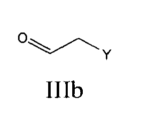 wherein Y has the same meaning as above;
with a thienyl zinc reagent optionally substituted on the thienyl ring, in the presence of a chiral ligand.

According to a further aspect, the present invention is directed to a process for the preparation of an enantiomerically enriched compound of formula V 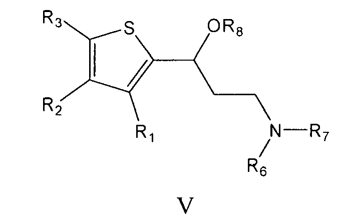 wherein
R₁, R₂, R₃, R₆ and R₇ are as defined above;
R₈ is selected from hydrogen, substituted or unsubstituted lower alkyl, hydroxyl protecting group, or substituted or unsubstituted aryl;
or a pharmaceutically acceptable salt, complex or solvate thereof,
which comprises,
a) an enantioselective addition reaction to a compound of formula III 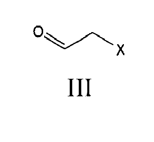
wherein X has the same meaning as above;
of a thienyl zinc reagent optionally susbtituted on the thienyl ring, in the presence of a chiral ligand to yield a compound of formula II 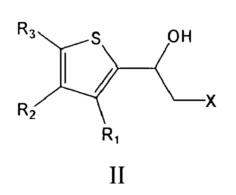 wherein,
R₁, R₂, R₃ and X are as defined above;
and further comprises, in any order, one or more of the steps selected from the group consisting of:
b) alkylation of the hydroxyl group;
c) amide formation;
d) reduction of amide to amine;
e) nucleophilic substitution;
e) hydroxyl , amide or amine protection;
f) hydroxyl , amide or amine deprotection;
g) salt, complex or solvate formation.

### DETAILED DESCRIPTION OF THE INVENTION

According to one aspect, the present invention relates to a process for the preparation of an enantiomerically enriched compound of formula II 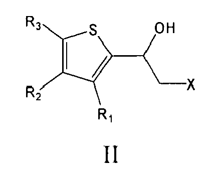 wherein,
R₁, R₂ and R₃ are each independently selected from hydrogen, halogen, substituted or unsubstituted lower alkyl or substituted or unsubstituted aryl;
X is -C(=O)-Z or Y, wherein Y is selected from -CH₂-OR₄, -CH₂-halogen or - CH₂-NR₆R₇;
wherein
Z is selected from -NR₆R₇ or -OR₅, wherein R₅ is selected from hydrogen, substituted or unsubstituted lower alkyl or ester activating group;
R₄ is selected from hydrogen, hydroxyl protecting group or hydroxyl activating group;
R₆ and R₇ are each independently selected from hydrogen, amino protecting group, amido protecting group or substituted or unsubstituted lower alkyl;
which comprises an enantioselective addition reaction to a compound of formula III 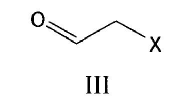 wherein X has the same meaning as above,
with a thienyl zinc reagent optionally substituted on the thienyl ring, in the presence of a chiral ligand.

Such a process gives the desired products of formula II with a high conversion and enantiomeric excess. This process has the further advantage that the zinc salts used or formed during the reaction are easily removed by aqueous work-up. The product of formula II is especially useful in the preparation of the enantiomers of the above mentioned thienylalcoxypropaneamines. Different compounds can be obtained depending on the substituents present on the thienyl ring.

We will discuss below the different reagents and conditions for the process of the invention.

### β-Substitutedaldehyde

The synthesis of β-substituted aldehydes of formula III which is the essential starting material for the synthesis according to the present invention, is well known to the person skilled in the art. For example, a compound of formula III wherein X is an ester group can be easily prepared through the methods described in US 4,749,811 or in Sato, Masayuki; et al, Synthesis, 1986, 8, 672-4.

A compound of formula III wherein X is -CH₂-OR₄, -CH₂-halogen or -CH₂-NR₆R₇, can be easily prepared through the methods described in Niederhauser, Andreas; et al, Helvetica Chimica Acta, 1973, 56(4), 1318-30. Synthesis, 7, 977-979; 1998. Angew. Chemie, International Edition in English, 6(5), 423-34; 1967. J. Org. Chem., 1997, 62(9), 2786-2797; J. Chem. Soc, Chemical Commu., (21), 1991, 1559-60.

### Thienyl zinc reagent

The thienylzinc reagent can be prepared *in situ* by a transmetallation reaction of a thienylboron reagent with dimethyl- or diethyl-zinc. Diethyl-zinc gives good results, although dimethyl-zinc is less prone to give alkylation reaction by-products in the reaction mixture with the aldehyde. The active species are presumably a mixed thienyl-ethyl-zinc or thienyl-methyl-zinc.

Among the suitable thienyl-boron reagents, thienylboronic acid, trithienylborane or 2-aminoethyl dithienylborinate depicted below: 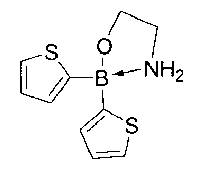 are preferably selected. More preferably, the thienyl-boron reagent is 2-aminoethyl dithienylborinate, because it can be made in higher purity and can be recrystallised from ethanol. Stable complexes of thienyl boranes are also preferred such as the NH₃ complex. The thienyl zinc can optionally have a R₁, R₂ and/or R₃ substituent as defined above for a compound of formula II.

### Chiral Ligand

With the aim of enantioselectively synthesizing a compound of formula (II) by an enantioselective addition reaction, the addition reaction must be carried out in the presence of a chiral catalyst or ligand, which forms the active catalyst *in situ* by reaction with the zinc reagent. That means that the ligand (or catalyst) must have at least one element of chirality such as one or more stereocentres or elements of planar chirality.

In principle, there is a great variety of N,O-, N,N-, N,S-, N,Se- or O,O-ligands that can be used in the process of the invention and all of them have to be in enantiomerically pure form. There are in the art about 600 known ligands for this type of reaction. Most of them can be found, for example in a recent review on catalytic asymmetric organozinc additions to carbonyl compounds [L. Pu, H.-B. Yu, Chem. Rev. 2001, 101, 757]. The nomenclature N,O-, N,N-, N,S-, N,Se- or O,O- refers to ligands that have at least these two coordinating heteroatoms.
It is obvious to the skilled person that in order to obtain a compound of formula II with the opposite configuration it is only necessary to use the enantiomer of the chiral ligands used in the present invention. Therefore, when a structure of a ligand is given throughout the present patent application, said structure is also meant to encompass the enantiomer of said ligand.

In a preferred embodiment of the present invention N,O-ligands and N,S-ligands are employed.

N,O-ligands are derived from β-amino alcohols and therefore have two carbon atoms between the heteroatoms. However, some of the ligands used in this reaction are those which present three carbon atoms between the heteroatoms. More preferably, the O is an alcohol.

In one embodiment a N,O-ligand having a structure-type (V) such as described below: 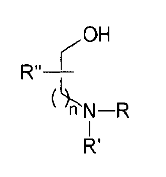 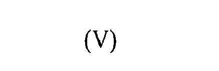 wherein n is 0 or 1, is used.

These ligands react with the zinc reagent forming a zinc-alcoxide complex which is more Lewis-acidic than the other present zinc species (reagent and product). Additionally, it is a Lewis-base catalyst (usually at the oxygen or sulfur atom). This zinc-alcoxide complex formed in situ is the active catalyst.

Typical ligands to be used in this addition reaction are the following compounds, their enantiomers, or derivatives thereof:

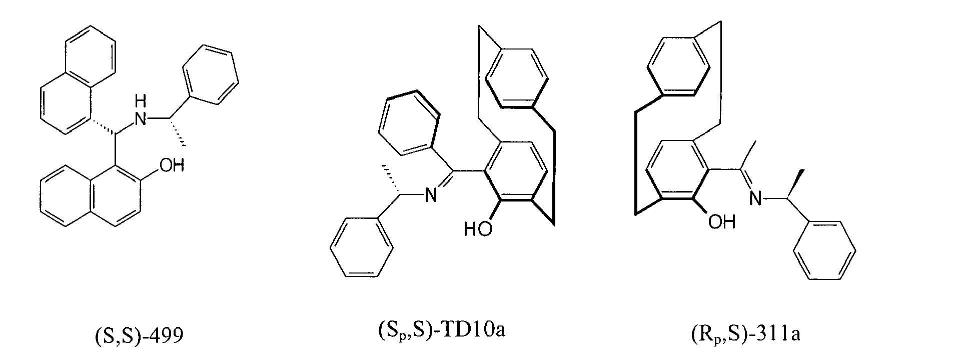

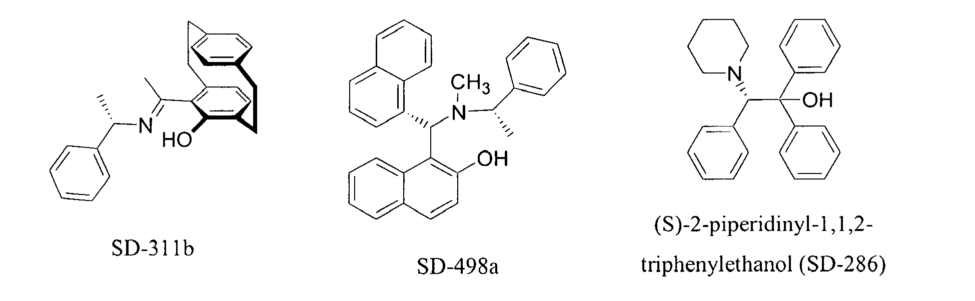

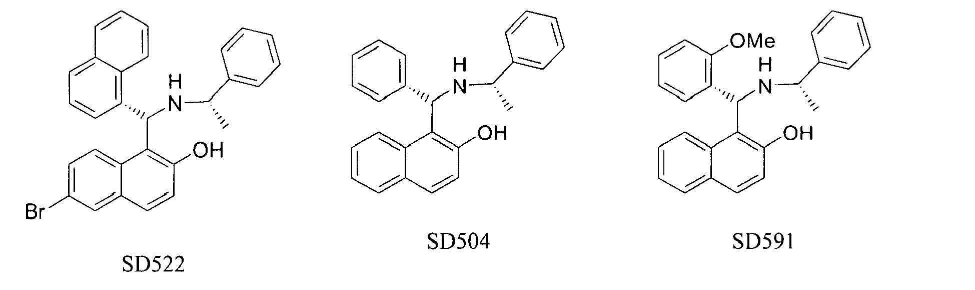

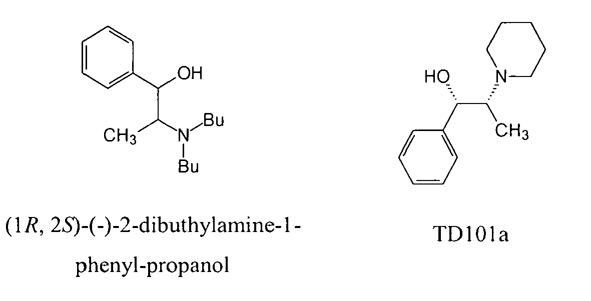

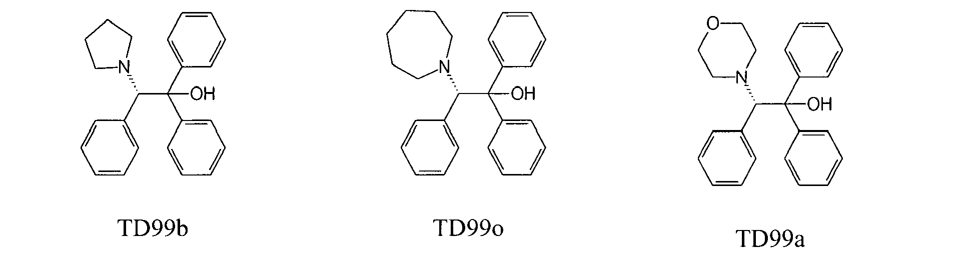

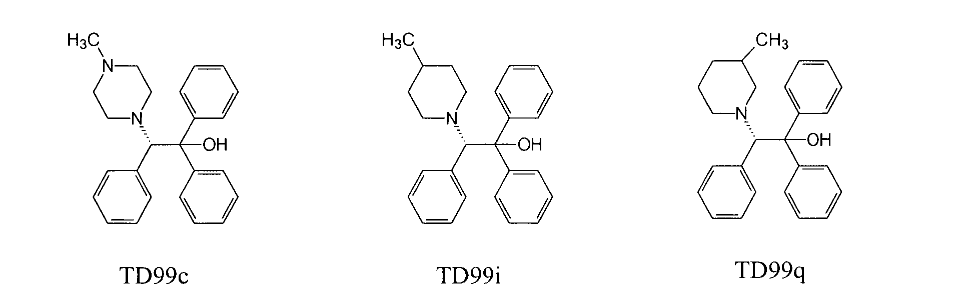

These ligands are available in both enantiomeric forms, allowing the selective synthesis of both enantiomers of the desired alcohol.

According to a further embodiment, the ligands are N,S- ligands, preferably selected from the group consisting of 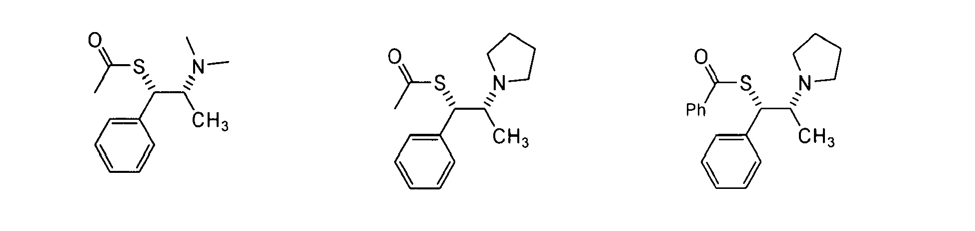 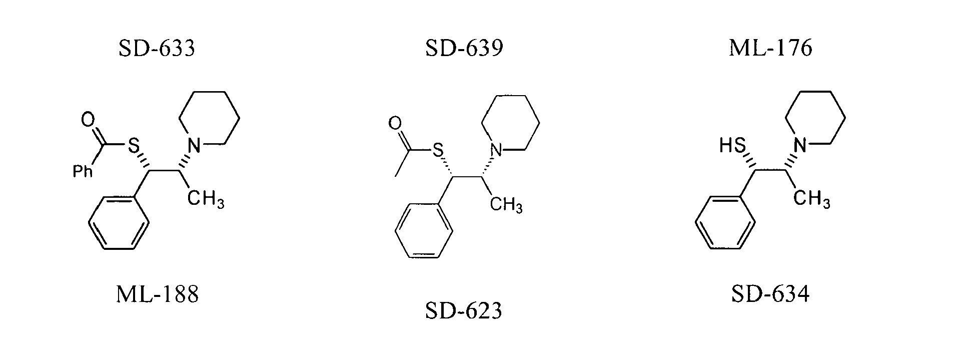 their enantiomers or derivates thereof.

N,S-ligands provided the desired product in good yield and remarkable enantiomeric excess. Among the N,S-ligands, those of formula VI 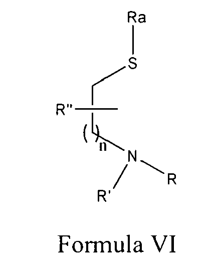 wherein Ra is selected from hydrogen or an alkoyl group, such as the aminothioacetates, are preferred. Ligands of formula VII are readily available through known procedures (see J. Kang, J. W. Lee, J. Kim, Chem. Commun. 1994, 17, 2009 or M.-J. Jin, S.-J. Ahn, K.-S. Lee, Tetrahedron Lett. 1996, 37, 8767.)

The reaction that takes place between the zinc reagent and the ligand of formula V leads to a complex of formula (VII): 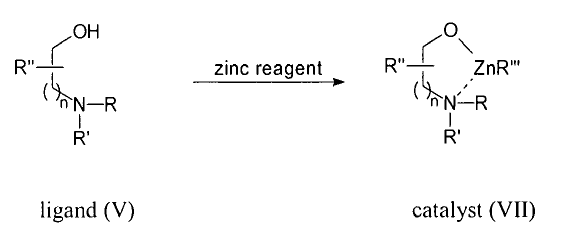 wherein n is 0 or 1 and R'" is thienyl, ethyl or methyl. A sulphur atom can be used instead of the oxygen atom, for example when using SD-623.

This zinc alkoxide complex (VII) is the active catalyst in the addition reaction which subsequently coordinates with the β-substitutedaldehyde in such a way that it induces the enantioselective addition of the thionyl group to said aldehyde. Without being bound by theory, it is believed that aminothiols and aminothioesters form similar complexes. However, the mechanism followed by aminothioesters complexes seems to be different from the mechanism followed by the intermediates of formula VII.

The concentration of the ligand should be low so as to reduce costs, but sufficient to provide good enantiomeric excess (ee). The ligands are preferably used in amounts of 0.1 to 100 mol%, more preferably 0.1 to 20 mol %. The use of more than the optimal amount of ligand is uneconomical and in some cases can lead to a lower selectivity. On the contrary, using less than optimal amount of ligand diminishes the selectivity due to a stronger influence of the non-catalysed and non-enantioselective background reaction.

### Solvent

Suitable solvents for the process of the invention are known from similar reactions and can be found in the above-mentioned references. Preferably they are non-coordinating hydrocarbons like e.g. pentane, hexane, heptane; aromatic solvents like benzene, toluene; chlorinated solvents like dichloromethane and 1,2-dichloroethane and weakly coordinating solvents like diethyl ether, methyl-*tert*-butyl ether (MTBE) and even polar coordinating solvents such as tiophene or dioxane. The most preferred solvents are toluene, hexane and heptane. These solvents allow the subsequent optional O-alkylation to be carried out in the same reaction mixture. In another variant of the process of the invention tiophene is used as the solvent, drastically improving yield and enantioselectivity and suppressing side-reactions such as the ethylation.

To perform the process, a mixture of the ligand and the compounds that form the zinc reagent can be prepared and stirred before the addition of the aldehyde. Usually, a pre-stirring is presumed to be beneficial for the selectivity because the deprotonation of the ligand by the zinc reagent giving the active catalyst requires a certain amount of time. However, under certain reaction conditions, it has been found that higher enantiomeric excess is achieved if short pre-stirring times are used. Once the aldehyde is added to the mixture of ligand and zinc reagent, the reaction time ranges between 1 h and 24 h.

The concentration of the aldehyde in the reaction is preferably low, such as between 0.01 molar and 2 molar. Although in some cases it has been observed that enantioselectivity increases at less concentrations, this is not suitable for an industrial process. In these cases a proper balance between enantioselectivity and adequate concentrations has to be found.

The process of the invention can be carried out at temperatures between -40 and 100°C. Preferably, temperatures between -20 and 20°C are used. The enantioselectivity of the reaction can also be dependent on the reaction temperature.

The process of the invention can also comprise the presence of additives, for example in order to improve the enantioselectivity by scavenging or complexing Lewis-acidic zinc salts present in the reaction or formed as products.

Suitable additives are for example alcohols, amines and derivatives of polyethylenglycol. More preferably the additive is selected from polyethylenglycols such as DiMPEG 1000, DiMPEG 2000, PEG 750, PEG 1000, PEG 2000, monoMPEG 2000 and PE-block-PEG, or from compounds such as 1,4-dioxane, *i*-propanol, triethylamine, tretramethylethylenediamine (TMEDA), imidazol, anisole, furane and thiophene. As mentioned above, the tiophene has the advantage of improving yield and enantioselectivity of the reaction, and can be used in quantities such that it acts also as a solvent.

In a preferred embodiment the process is directed to the synthesis of each of the following alcohols of formula II with the highest possible enantiomeric purity: 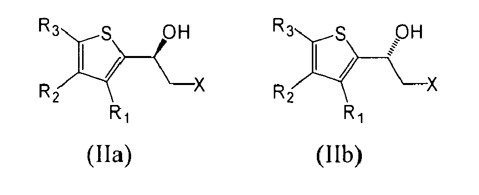 wherein R₁, R₂ and R₃ are as defined above.

The zinc salts used are easily removed by aqueous work-up and the obtained alcohol can be purified through chromatography or crystallization. Alternatively, the alcohol can be advantageously used without further purification in the next step, which can be carried out in the same reaction medium.

Thus, according to another aspect, the invention relates to a process as defined above which further comprises the step of O-alkylation of an enantiomerically enriched compound of formula (II). Preferably, oxygen is alkylated with a naphtalene derivative of formula IV 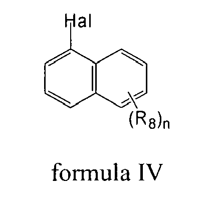 wherein Hal is F, Cl, Br or I; R₈ is substituted or unsubstituted lower alkyl or substituted or unsubstituted aryl; and n is comprised between 0 and 7. This process has been described in the art for the synthesis of duloxetine. The most frequently used method is the alkylation of the alcohol with 1-fluoronaphtalene in the presence of strong base (see EP 654264 or EP 650965, for example).

According to a preferred embodiment the O-alkylation is carried out on the product of the process of the invention without an intermediate separation or purification step.

The alkylation is preferably carried out directly in the same reaction medium resulting from the process of the invention, without further purification of the carbinol. Besides being more economical, this direct alkylation avoids racemisation of the compound of formula (II) during workup of the addition reaction according to the present invention, which has been observed under certain reaction conditions.
According to a further aspect, the present invention is directed to a process for the preparation of an enantiomerically enriched compound of formula V 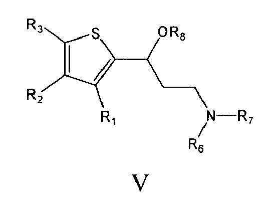 wherein
R₁, R₂, R₃, R₆ and R₇ are as defined above;
R₈ is selected from hydrogen, substituted or unsubstituted lower alkyl,
hydroxyl protecting group or substituted or unsubstituted aryl;
or a pharmaceutically acceptable salt, complex or solvate thereof,
which comprises,
a) an enantioselective addition reaction to a compound of formula III 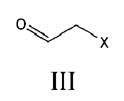 wherein X has the same meaning as above;
   of a thienyl zinc reagent optionally susbtituted on the thienyl ring, in the presence of a chiral ligand to yield a compound of formula II 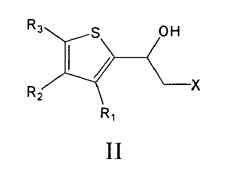 wherein,
   R₁, R₂, R₃ and X are as defined above;
   and further comprises in any order one or more of the steps selected from the group consisting of:
b) alkylation of the hydroxyl group;
c) amide formation;
d) reduction of amide to amine;
e) nucleophilic substitution;
e) hydroxyl , amide or amine protection;
f) hydroxyl , amide or amine deprotection;
g) salt, complex or solvate formation.
Preferably, the compound of formula V is an enantiomerically enriched form of N-methyl-N-[3-(naphthalene-1-yloxy)-3-(2-thienyl)propyl]amine, more preferably, in the form of a hydrochloride salt.
The term "enantiomerically enriched compound " refers to a compound that has at least one chiral center and in which there is excess of one enantiomer over the other. Thus it does not comprise the racemic mixture. For a mixture of (+)- and (-)-enantiomers, with composition given as the mole or weight fractions *F*(+) and *F*(-) (where *F*(+) + *F*(-) = 1) the enantiomer excess is defined as |*F*(+)-*F*(-)|. (and the percent enantiomer excess by 100|*F*(+)-*F*(-)|). Frequently this term is abbreviated as e.e. Enantiomerically enriched compound means that the e.e is not 0. In the present invention it is preferred that the e.e is above 505, more preferably above 60%, even more preferably above 70% or 80%, and most preferably above 90% or 95%.

The term "lower alkyl" refers to a linear or branched hydrocarbon chain which contains about 1 to 5 carbon atoms such as methyl, ethyl, propyl, isopropyl, butyl, isobutyl, sec-butyl, tert-butyl, pentyl, etc.

By "thienyl zinc reagent optionally substituted on the thienyl ring", we refer to a thienyl zinc reagent which can be substituted at 2, 3, 4 or 5 position of the thienyl ring by an halogen, a lower alkyl or an aryl group.

"halogen" refers to fluorine, chlorine, bromine or Iodine.

"Aryl" refers to an aromatic hydrocarbon radical such as phenyl, naphthyl or anthracyl. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.

"Hydroxyl protecting group" refers to a group that blocks the OH function for further reactions and can be removed under controlled conditions. The hydroxyl protecting groups are well known in the art, representative protecting groups are silyl ethers such as trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether; alkyl ethers such as methyl ether, tert-butyl ether, benzyl ether, p-methoxybenzyl ether , 3,4-dimethoxybenzyl ether, trityl ether; allyl ether; alkoxymethyl ether such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers; methylthiomethyl ether; Esters such as acetate ester, benzoate ester; pivalate ester; methoxyacetate ester; chloroacetate ester; levulinate ester; Carbonates such as benzyl carbonate, p-nitrobenzyl carbonate, tert-butyl carbonate, 2,2,2-trichloroethyl carbonate, 2-(trimethylsilyl)ethyl carbonate, allyl carbonate; and sulphates such as SO₃.py . In some cases activating groups such as triflate are also included as protecting groups. Additional examples of hydroxyl protecting groups can be found in reference books such as Greene and Wuts' "Protective Groups in Organic Synthesis", John Wiley & Sons, Inc., New York, 1999.

"Amino protecting group" refers to a group that blocks the NH₂ function for further reactions and can be removed under controlled conditions. The amino protecting groups are well known in the art, representative protecting groups are carbamates and amides such as substituted or unsubstituted or substituted acetates. Also different alkyl moeties may serve as amino protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amino protecting groups can be found in reference books such as Greene and Wuts' "Protective Groups in Organic Synthesis", John Wiley & Sons, Inc., New York, 1999.

"Amido protecting group" refers to a group that blocks the -C(=O)NH₂ function for further reactions and can be removed under controlled conditions. The amido protecting groups are well known in the art, representative protecting groups are carbamates such as substituted or unsubstituted acetates. Also different alkyl moeties may serve as amido protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amido protecting groups can be found in reference books such as Greene and Wuts' "Protective Groups in Organic Synthesis", John Wiley & Sons, Inc., New York, 1999.

"ester activating group" referrers to a group which increases the reactivity of the ester functionality.

"hydroxyl activating group" referrers to a group which increases the reactivity of the hydroxyl functionality.

References herein to substituted groups in the compounds of the present invention refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C1-6 alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

## Claims

1. A process for the preparation of an enantiomerically enriched compound of formula II 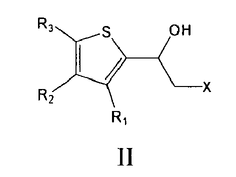 wherein,
R₁, R₂ and R₃ are each independently selected from hydrogen, halogen, substituted or unsubstituted lower alkyl or substituted or unsubstituted aryl;
X is -C(=O)-Z or -Y, wherein -Y is selected from -CH₂-OR₄, -CH₂-halogen or - CH₂-NR₆R₇;
wherein
Z is selected from -NR₆R₇ or -OR₅, wherein R₅ is selected from hydrogen, substituted or unsubstituted lower alkyl or ester activating group;
R₄ is selected from hydrogen, hydroxyl protecting group or hydroxyl activating group;
R₆ and R₇ are each independently selected from hydrogen, amino protecting group, amido protecting group or substituted or unsubstituted lower alkyl;
which comprises an enantioselective addition reaction to a compound of formula III 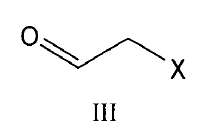 wherein X has the same meaning as above;
with a thienyl zinc reagent optionally substituted on the thienyl ring, in the presence of a chiral ligand.

2. A process according to claim 1 for the preparation of an enantiomerically enriched compound of formula IIa 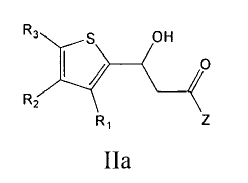 wherein,
R₁, R₂ and R₃ and Z have the same meaning as in claim 1,
which comprises an enantioselective addition reaction to a compound of formula IIIa 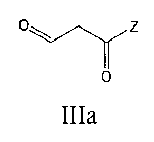 wherein Z has the same meaning as in claim 1;
with a thienyl zinc reagent optionally susbtituted on the thienyl ring, in the presence of a chiral ligand.

3. A process according to claim 1 for the preparation of an enantiomerically enriched compound of formula IIb 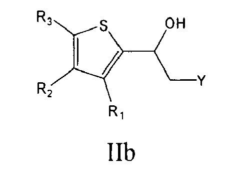 wherein,
R₁, R₂ and R₃ and Y have the same meaning as in claim 1;
which comprises an enantioselective addition reaction to a compound of formula IIIb 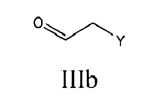 wherein -Y has the same meaning as in claim 1;
with a thienyl zinc reagent optionally susbtituted on the thienyl ring, in the presence of a chiral ligand.

4. A process according to any of the preceding claims, wherein R₁, R₂ and R₃ are hydrogen.

5. A process according to claim 1 wherein the thienyl zinc reagent is prepared in situ by a transmetallation reaction of a thienylboron reagent with dimethyl-zinc or diethyl-zinc.

6. A process according to claim 5 wherein the thienylboron reagent is selected from thienylboronic acid, trithienylborane or 2-aminoethyl dithienylborinate depicted below:

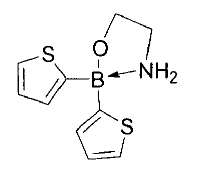

7. A process according to claim 1 wherein the chiral ligand is a N,O-, N,N-, N,S-, N,Se- or O,O-ligand in its enantiomerically pure form.

8. A process according to claim 7 wherein the chiral ligand is a N,O-ligand, most preferably the O is an alcohol.

9. A process according to claims 8 wherein the N,O-ligands are selected from the following compounds: 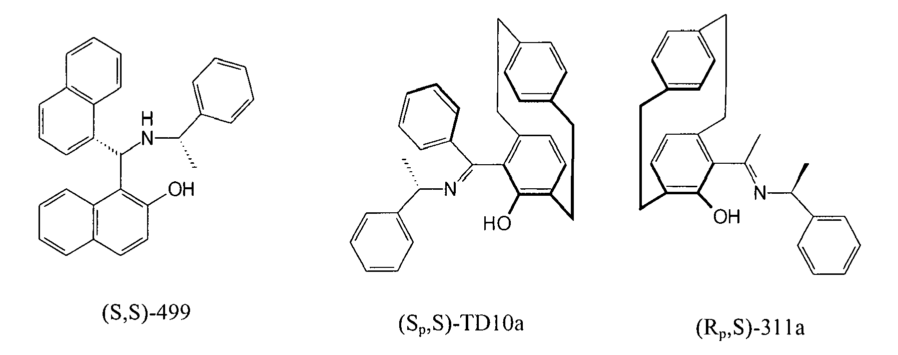 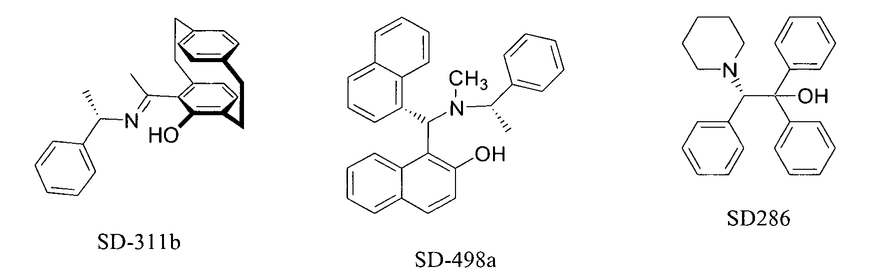 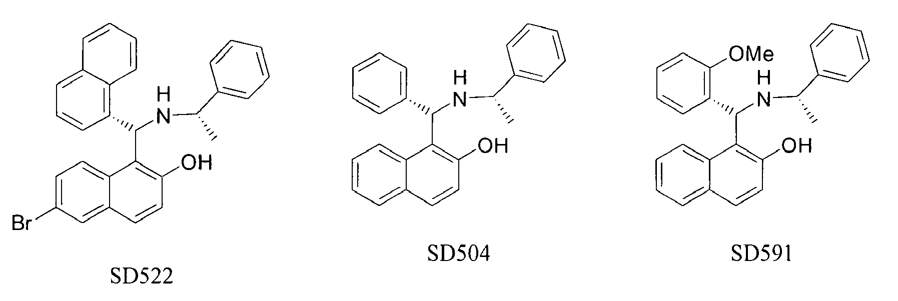 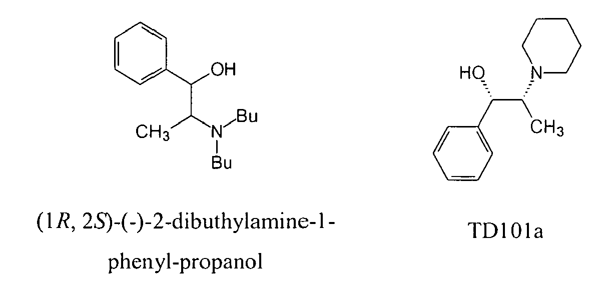 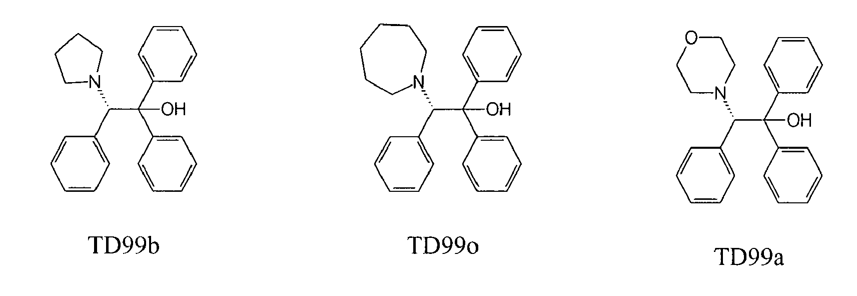 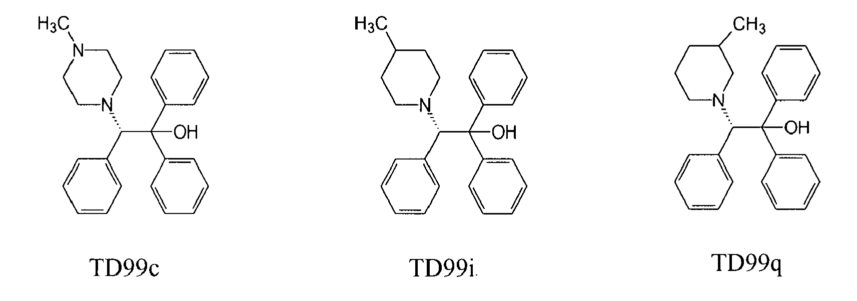 or an enantiomer thereof.

10. A process according to claim 7 wherein the ligand is a N,S-ligand.

11. A process according to claim 10, wherein the ligand is selected from the compounds having the following formulas 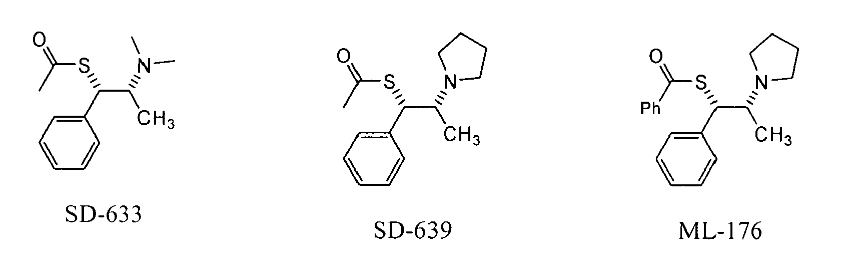 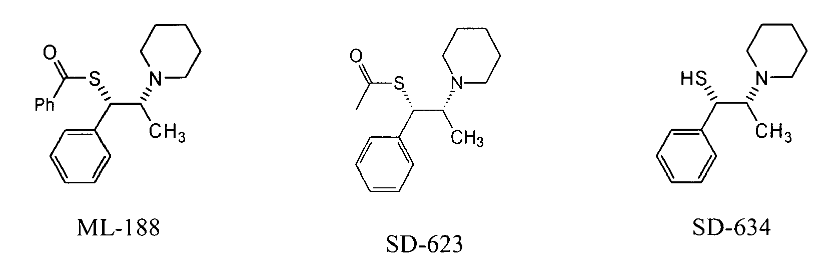 or the enantiomers of the same.

12. A process according to anyone of claims 1-11 wherein the ligands are used in amounts ranged between 0.1 and 20 mol%.

13. A process according to anyone of claims 1-12 wherein the temperature is comprised between -20°C and 20°C, preferably -10°C to 10°C.

14. A process according to anyone of claims 1-13 wherein the aldehyde concentration ranges between 0.01 molar and 2 molar.

15. A process according to anyone of claims 1-14 wherein the solvent is non-coordinating, chlorinated or weakly coordinating.

16. A process according to claim 1 which further comprises an O-alkylation of the enantiomerically enriched compound of formula II.

17. A process according to claim 16, which comprises an O-alkylation with a naphtalene derivative of formula IV 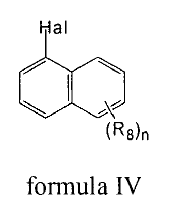 wherein Hal is F, Cl, Br or I; R₈ is substituted or unsubstituted lower alkyl or substituted or unsubstituted aryl; and n is comprised between 0 and 7.

18. A process according to any of claims 16 and 17 wherein the O-alkylation is carried out on the product of the process as defined in anyone of claims 1-15, without an intermediate separation or purification step.

19. A process for the preparation of an enantiomerically enriched compound of formula V 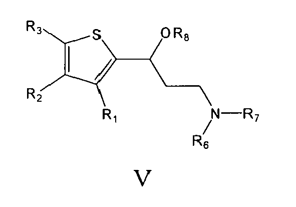 wherein
R₁, R₂, R₃, R₆ and R₇ are as defined in claim 1;
R₈ is selected from hydrogen, substituted or unsubstituted lower alkyl, hydroxyl protecting group or substituted or unsubstituted aryl;
or a pharmaceutically acceptable salt, complex or solvate thereof,
which comprises,
a) an enantioselective addition reaction to a compound of formula III 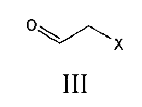 wherein X has the same meaning as in claim 1;
of a thienyl zinc reagent optionally substituted on the thienyl ring, in the presence of a chiral ligand to yield a compound of formula II 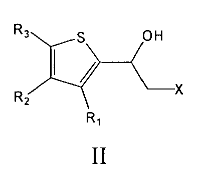 wherein,
R₁, R₂, R₃ and X are as defined in claim 1;
and further comprises in any order one or more of the steps selected from the group consisting of:
b) alkylation of the hydroxyl group;
c) amide formation;
d) reduction of amide to amine;
e) nucleophilic substitution;
e) hydroxyl , amide or amine protection;
f) hydroxyl , amide or amine deprotection;
g) salt, complex or solvate formation.

20. Process according to claim 19, wherein the compound of formula V is an enantiomerically enriched form of N-methyl-N-[3-(naphthalene-1-yloxy)-3-(2-thienyl)propyl]amine.

21. Process according to any of claims 19 and 20, wherein the compound of formula V is in the form of a hydrochloride salt.
